# FASCICULE DE BREVET EUROPEEN

(11) **EP 2 959 936 B1**
(45) Date de publication et mention de la délivrance du brevet: **31.03.2021**
(21) Numéro de dépôt: 15167098.1
(22) Date de dépôt: 11.05.2015
(51) Int. Cl.: A61N 1/05

(54) **CAPSULE INPLANTABLE À FIXATION PAR VISSAGE, NOTAMMENT CAPSULE AUTONOME DE STIMULATION CARDIAQUE**
IMPLANTIERBARE KAPSEL MIT FIXIERUNG DURCH VERSCHRAUBUNG, INSBESONDERE AUTONOME HERZSTIMULATIONSKAPSEL
IMPLANTABLE CAPSULE WITH ATTACHMENT BY SCREWING, IN PARTICULAR AN AUTONOMOUS CARDIAC STIMULATION CAPSULE

(30) Priorité: 25.06.2014 FR 1455896
(43) Date de publication de la demande: 30.12.2015
(73) Titulaire: Sorin CRM SAS, 92140 Clamart Cedex (FR)
(72) Inventeur: Regnier, Willy, 91160 LONGJUMEAU (FR); D'Hiver, Philippe, 92320 CHATILLON (FR); Ollivier, Jean-François, 91190 VILLIERS LE BACLE (FR)
(74) Mandataire: Grünecker Patent- und Rechtsanwälte PartG mbB

(56) Documents cités:
- WO-A1-2012/051235
- WO-A2-01/26706
- US-A1- 2008 109 042

## Description

L'invention concerne les "dispositifs médicaux implantables actifs" tels que définis par la directive 90/385/CEE du 20 juin 1990 du Conseil des communautés européennes, plus précisément les implants permettant de surveiller en continu le rythme cardiaque et délivrer si nécessaire au cœur des impulsions électriques de stimulation, de resynchronisation et/ou de défibrillation en cas de trouble du rythme détecté par le dispositif.

L'invention concerne notamment, mais de manière non limitative, ceux de ces dispositifs qui se présentent sous forme d'une capsule autonome destinée à être implantée dans une cavité cardiaque (ventricule ou oreillette, à droite ou à gauche). Ces capsules sont dépourvues de toute liaison mécanique à un dispositif principal implanté (tel qu'un boitier de générateur d'impulsions de stimulation) ou non implanté (périphérique externe tel que programmateur ou dispositif de *monitoring* pour le suivi à distance du patient), et sont dénommées pour cette raison "capsules *leadless*"*,* pour les distinguer des électrodes ou des capteurs disposés à l'extrémité distale d'une sonde (*lead*) conventionnelle, qui est parcourue sur toute sa longueur par un ou plusieurs conducteurs reliant par voie galvanique l'électrode ou le capteur à un générateur connecté à une extrémité opposée, proximale, de la sonde.

Le EP 2 394 695 A1 (Sorin CRM) décrit une capsule intracardiaque autonome, ainsi qu'une procédure permettant de l'implanter au site de détection/stimulation choisi et de la repositionner si nécessaire.

On notera toutefois, comme on le comprendra à la lecture de la description, que le caractère autonome de la capsule n'est pas intrinsèquement une caractéristique nécessaire de l'invention, et que cette dernière peut être aussi bien appliquée à des capsules montées de façon permanente à l'extrémité distale d'une sonde.

Une capsule implantable comprend un corps abritant les principaux éléments du dispositif (circuits électroniques, source d'énergie, électrodes de stimulations, etc.) et une embase solidaire du corps et supportant rigidement un moyen de fixation à la paroi.

Dans le cas des sondes cardiaques, deux types de fixation sont reconnus et traditionnellement employés : la fixation dite à barbes est la plus ancienne et encore marginalement utilisée, mais les sondes basées sur une vis de fixation ont supplanté les sondes à barbes et représentent actuellement la majorité du marché. Elles permettent une fixation généralement robuste et efficace. La vis est une vis hélicoïdale saillante prolongeant axialement le corps de la capsule et destinée à pénétrer dans le tissu cardiaque par vissage au site d'implantation, de la même manière que pour les sondes à vis conventionnelles.

Toutefois, la fixation de tels dispositifs reste un point critique dans la mesure où un détachement accidentel de la capsule amènerait cette dernière à être libérée dans la cavité cardiaque puis transportée par le sang dans le système veineux ou artériel. Le risque de complication pour le patient serait alors extrêmement élevé, ainsi que le risque de blessure du système cardiaque qui peut être engendré par l'extrémité du système de fixation ou toutes autres zones saillantes de l'implant comme par exemple une électrode pointue ou une arête saillante.

Davantage qu'un dispositif à sonde, un dispositif autonome subit quant à lui les contraintes et les mouvements engendrés par la paroi cardiaque, car il ne bénéficie pas de l'effort axial de maintien de la part du corps de sonde.

Pour remplir sa fonction d'ancrage permanent, le système de fixation doit donc comporter également une fonction d'irréversibilité, c'est-à-dire qu'il ne pourra être retiré de la paroi cardiaque que par l'intervention volontaire du médecin et suivant une procédure prédéfinie, mais en aucun cas par les mouvements ou vibrations répétés du cœur, ou par la modification du muscle cardiaque du faite de la maladie ou du vieillissement des tissus.

Le WO 2012/051235 A1 décrit des moyens de prévention de la rotation inverse qui mettent en œuvre des aménagements avec des pointes saillantes formées directement sur la vis et orientées dans une direction circonférentielle opposée au sens de vissage, ou encore des pointes saillantes pénétrant dans les tissus à la base de la vis. Cela implique des dommages importants pour tous les tissus traversés lors de la pose de l'implant ou avoisinants

La présente invention vise à proposer un dispositif autonome implantable qui permette d'assurer une bonne opposition au dévissage, tout en ayant un effet traumatique beaucoup plus réduit.

Cet aspect est particulièrement critique, dans la mesure où les tissus avoisinants sont les premières cibles du processus de stimulation, et il est primordial de protéger ces tissus lors de la pose et pendant le fonctionnement du dispositif, afin d'assurer des seuils de stimulation bas et donc préserver la longévité du dispositif.

Plus précisément, l'invention propose à cet effet une capsule implantable, notamment une capsule autonome de stimulation cardiaque, comprenant de manière en elle-même connue un corps tubulaire pourvu à son extrémité distale d'un élément d'ancrage à vis hélicoïdale apte à pénétrer dans un tissu d'une paroi d'un organe d'un patient, le corps abritant un ensemble d'éléments fonctionnels de la capsule. La capsule comprend en outre un ensemble de pointes orientées dans une direction circonférentielle opposée à celle du vissage.

De façon caractéristique de l'invention, la capsule comprend : une face d'appui annulaire formée dans une région annulaire entourant la base de la vis et située à l'extérieur de l'organe d'ancrage en direction radiale ; et, formés de manière affleurante dans ladite face d'appui annulaire, des aménagements en creux définissant ledit ensemble de pointes.

Selon diverses caractéristiques subsidiaires avantageuses :
- les aménagements en creux comprennent des évidements obliques formés à partir d'une face annulaire généralement continue du support de façon à former des pointes dirigées dans une direction circonférentielle opposée au vissage, avec notamment un angle d'inclinaison des évidements compris entre 40 et 60° par rapport à la surface de la face annulaire ;
- la proportion de la superficie de la face annulaire occupée par les évidements est comprise entre 20 et 40 %, et préférentiellement voisine de 30 % ;
- l'organe d'ancrage à vis est formé à partir d'un fil métallique élastique enroulé en hélice et se terminant en pointe, et apte à générer un effort de traction de ladite région annulaire en direction de ladite paroi, notamment avec un espacement entre les spires successives de l'hélice qui augmente entre une base de la vis et sa pointe ;
- la capsule comprend en outre une électrode en pointe en saillie à l'intérieur de l'organe à vis, cette électrode étant configurée en harpon. L'électrode peut notamment être une pièce de révolution centrée sur l'axe de rotation de la capsule lors du vissage, et/ou comprendre une tête conique à l'extrémité libre d'une tige plus étroite que la tête ;
- la capsule comprend en outre un anneau de diffusion de produit pharmaceutique disposé à l'intérieur de la région annulaire, les évidements étant aptes à favoriser la circulation du produit vers l'extérieur de ladite région ;
- la capsule comprend une pièce en matériau biodégradable dans laquelle sont réalisés les aménagements en creux définissant l'ensemble de pointes, le matériau biodégradable pouvant notamment être un biopolymère bioabsorbable du groupe formé par : les polylactides PLA, les polyglycolides PGA et les polylactides-co-glycolides PLGA.

On va maintenant décrire un exemple de mise en œuvre de la présente invention, en référence aux dessins annexés où les mêmes références désignent d'une figure à l'autre des éléments identiques ou fonctionnellement semblables.
La Figure 1 est une vue d'ensemble en perspective d'une capsule implantable selon l'invention.
La Figure 2 est une vue en perspective et en demi-coupe d'une région distale de la capsule de la Figure 1.
La Figure 3 est une vue en perspective d'un support de vis d'ancrage de la capsule des Figures 1 et 2.
La Figure 4 est une vue en perspective d'un détail du support de la Figure 3.
La Figure 5 est une vue de profil du détail de la Figure 4.
La Figure 6 est une vue en perspective et en demi-coupe d'une région distale d'une capsule incorporant une variante de réalisation.
La Figure 7 est une vue en perspective, selon une autre orientation, de cette même région distale.
La Figure 8 est une vue en perspective d'un ensemble vis-support selon un autre aspect d'une capsule implantable, la vis étant visible en transparence.
La Figure 9 est une vue en perspective de l'ensemble vis-support de la Figure 8.
La Figure 10 est une vue partielle en perspective d'une variante d'aménagement du support pour la fixation de la vis sur le support.

On va maintenant décrire des exemples de réalisation d'une capsule implantable.

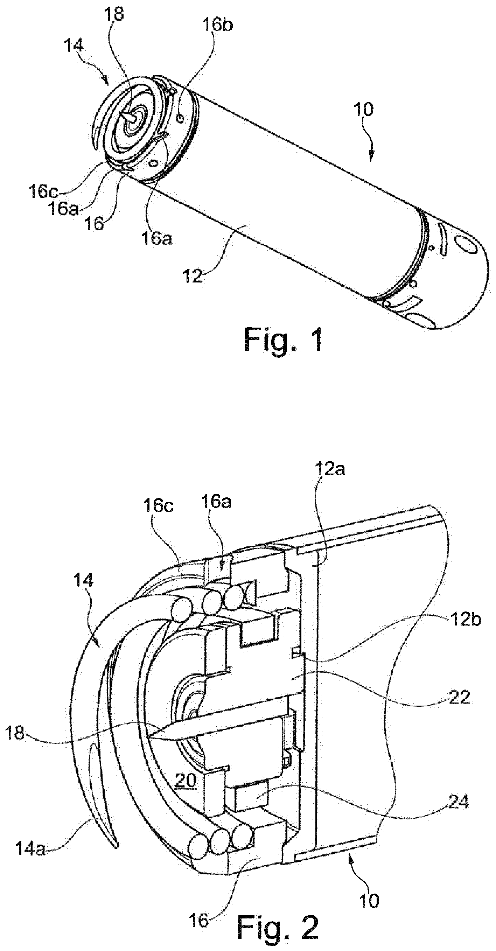

En référence tout d'abord à la Figure 1, on a représenté une capsule implantable 10, ici une capsule autonome de stimulation cardiaque, comprenant un corps de capsule 12 et un organe d'ancrage de type vis hélicoïdale 14.

La vis 14 est formée par un fil enroulé en hélice à pas de vissage à droite et est montée sur un support de vis 16 intégrant des aménagements assurant l'irréversibilité de l'ancrage comme on va le voir dans la suite.

La vis 14 est rendue définitivement solidaire de son support 16 par exemple par soudage laser selon les points de soudure répartis. L'ensemble est ensuite solidarisé définitivement au corps de la capsule 12 par un cordon de soudure laser.

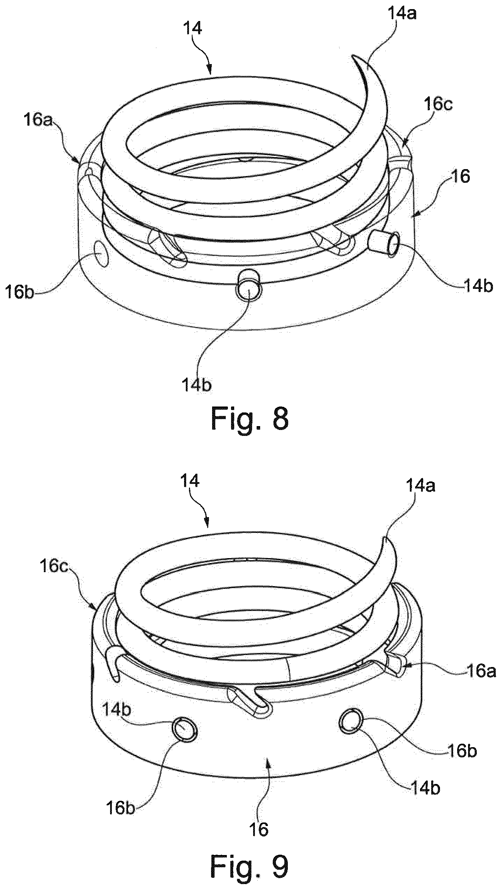

On notera que les matériaux de la vis 14 et de son support 16 peuvent être différents, par exemple un couple platine/iridium 90/10 pour la vis 14 et du titane pour le support 16. Il est en effet possible, comme on le verra plus bas en référence au mode de réalisation illustré Figures 8 et 9, de mettre en œuvre une méthode d'assemblage efficace de ces deux éléments même s'ils sont réalisés dans des matériaux hétérogènes.

Les différentes pièces sont par exemple réalisées en alliages métalliques biocompatibles tels qu'un acier inoxydable ou un alliage de titane, de préférence un alliage de titane biocompatible pour le corps de capsule 12 et le support 16.

Les aménagements pour l'irréversibilité de la fixation comprennent des encoches ou évidements 16a formés dans le support 16.

L'ensemble de fixation constitué des pièces 14, 16 présente un diamètre de préférence égal à celui du corps de l'implant, typiquement de 6 à 7 mm, et une longueur axiale légèrement inférieure, typiquement entre 4 et 6 mm. La vis 14 délimite un espace libre occupé par un autre sous-ensemble du dispositif, ici un sous-ensemble d'électrodes de stimulation. Le système de fixation est conçu pour fixer l'implant de façon stable et durable dans le temps grâce à la vis hélicoïdale 14 formant un ressort à spires évolutives et se terminant par une pointe apte à perforer l'endothélium et à pénétrer dans les tissus musculaires, de façon à plaquer la paroi cardiaque sur la face frontale généralement annulaire du support 16, sensiblement à la même position (en direction axiale) que la surface d'appui interne du système d'électrode.

Plus en détail, le support de vis 16 présente sur ladite face frontale 16c une série d'évidements 16a qui réalisent la fonction d'irréversibilité une fois en contact avec l'endothélium. Dans cette position, le système de fixation est indémontable : sous l'action de la vis 14 qui agit comme un ressort de traction réalisant un effort de retenue axial, la paroi cardiaque est plaquée contre la face 16c du support de vis 16 et s'ancre localement dans les évidements (ici au nombre de six, régulièrement répartis) par l'effet ressort précité.

Comme on va le voir plus en détail dans la suite, les six évidements définissent autant d'arêtes vives, mais peu profondes, dans leur région (en direction circonférentielle) opposée au sens de vissage de l'hélice et réalisent alors six points d'ancrage ponctuels et répartis sur l'endothélium (espacement mutuel de 60° dans cet exemple), qui assurent une irréversibilité du vissage réalisé pour ancrer la capsule.

Maintenant plus en détail et en référence à la Figure 2, on observe un sous-ensemble de stimulation constitué ici par deux électrodes 18 et 20 maintenues par un support d'électrodes 22 qui supporte également à sa périphérie un anneau 24 de diffusion d'un produit stéroïde.

Le support 22 est positionné coaxialement au support de vis 16, à l'intérieur de celui-ci, et fixé par collage ou tout autre moyen approprié sur une paroi 12a fermant l'espace intérieur du corps de capsule 12 à son extrémité distale côté vis, le support étant localisé précisément grâce à un épaulement circulaire 12b prévu sur la face extérieure de ladite paroi. L'électrode de stimulation centrale en pointe 18 est fixée (par sertissage, collage, etc.) au centre du support 22 et connectée électriquement à l'électronique interne de la capsule logée dans le corps 12, par l'intermédiaire d'une micro-traversée. La deuxième électrode 20 présente ici la forme d'une rondelle positionnée et fixée, par exemple par collage, sur la face externe du support 22 et est connectée également par le biais d'une micro-traversée à l'électronique interne.

L'anneau 24 est imprégné d'un produit stéroïde tel que la dexaméthasone et est positionné sous l'électrode 20. Le produit stéroïde permet de réduire l'inflammation tissulaire durant les premières semaines qui suivent l'implantation.

L'électronique associée aux électrodes permet de mettre en œuvre, dans le cas d'une stimulation cardiaque, des fonctions de détection et de stimulation, la structure décrite garantissant un contact fiable et permanent entre les électrodes 18, 20 et les tissus.

La vis en hélice 14 est ici constituée d'un fil métallique d'un diamètre de l'ordre de 0,5 mm, avec un diamètre d'enroulement typiquement 5 mm environ et de préférence identique à celui du corps 12 du dispositif. La vis comprend une base plane suivie par deux spires jointives et une spire finale s'étendant par exemple sur environ 1,5 tour avec un espace inter-spire du même ordre de grandeur que le diamètre du fil. L'extrémité libre de la vis 14 est affinée, dans le cas d'espèce par deux usinages dans des plans mutuellement orthogonaux, créant ainsi une pointe 14a perforante mais non tranchante. Le but de cette pointe est de permettre de traverser facilement l'endothélium puis de pénétrer dans le muscle cardiaque tout en créant un minimum de lésions tissulaires. La vis 14 est ici réalisée en acier inoxydable 316L implantable et biocompatible ou toute autre matière équivalente, qui délivre une raideur d'environ 0,1N/mm (raideur linéaire du ressort, mesurée par traction ou compression à l'aide d'un dynamomètre sur une course de 1 mm).

Ceci donne à la vis une souplesse axiale qui lui confère un effet ressort, opérant un effort de traction assurant un contact ferme entre le support 16 et la paroi cardiaque. Ainsi lors de la pénétration de la vis 14 dans le muscle, la vis se déforme axialement jusqu'au contact du bord libre 16c du support 16 avec l'endothélium. L'effet ressort de la vis va alors comprimer axialement l'endothélium et le muscle entre les spires et créer un effet de coincement. De plus, la proximité immédiate des spires et leur effet de traction sur l'endothélium forcent l'entrée de ce dernier dans les encoches d'antidévissage. Au cours de ce mouvement, l'électrode de stimulation en pointe 18 perfore l'endothélium et vient ainsi en contact avec les cellules excitables de la paroi cardiaque.

D'autres configurations de spires sont bien entendu possibles, mais il est avantageux de prévoir un espacement entre spires qui augmente entre la base de la vis (où comme on l'a vu l'espacement peut être nul) et la pointe 14a de la vis. Ceci permet de favoriser l'effort de traction axial appliquant le support 16 contre la paroi cardiaque.

On comprend par ailleurs que l'électrode 18 contribue à la fixation de la capsule tout en assurant contact fiable et continu avec les tissus comprimés grâce à l'effort axial de la vis 14 en direction du support 16.

Une telle configuration est particulièrement adaptée pour une stimulation basse énergie, avec une longueur d'aiguille de l'ordre de 1,2 mm et un diamètre de l'ordre de 0,4 mm, soit une surface active de l'ordre de 1 mm².

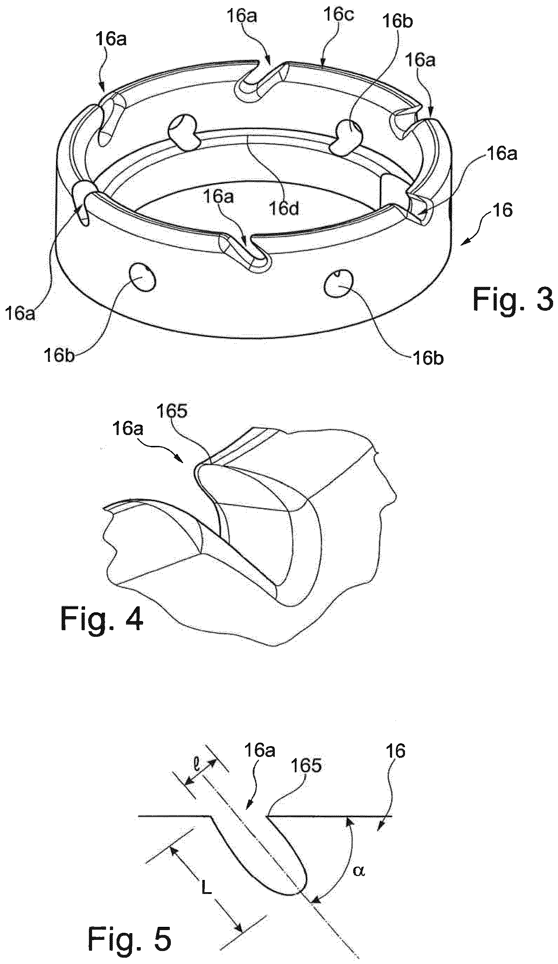

En référence tout particulièrement aux Figures 3 à 5, le support 16 de la vis 14 présente comme on l'a indiqué une face ou bord d'appui 16c, de préférence de profil légèrement arrondi, à partir duquel sont formés, en direction oblique, six évidements 16a permettant de réaliser six arêtes saillantes 165 très localisées et très ponctuelles (voir notamment Figures 4 et 5) assurant l'irréversibilité du vissage de la capsule réalisé comme décrit précédemment. La perforation localisée de l'endothélium est obtenue par les arêtes saillantes 165, les autres arêtes qui forment les limites de l'encoche étant volontairement très arrondies afin de ne pas propager ni agrandir le percement de l'endothélium lors des efforts de dévissage. La surface d'appui supportant les contraintes de dévissages étant maximisée, les risques de déchirements de l'endothélium sont minimisés. Préférentiellement mais non limitativement, la surface relative occupée par les évidements de la face d'appui 16c représente de 20 à 40 % de cette surface, et plus préférentiellement autour de 30 %, ce qui permet de limiter l'effet traumatique des arêtes saillantes 165 en limitant la pénétration des tissus dans les évidements 16a, tout en assurant un bon accrochage desdites arêtes 165 dans l'endothélium, et donc l'efficacité de la fonction antidévissage.

Les dimensions des évidements peuvent varier. Par exemple et en référence particulièrement à la Figure 5, leur largeur ℓ peut être de l'ordre de 0,3 à 0,8 mm, leur profondeur ou longueur L peut être comprise entre 0,5 et 1 mm, et leur inclinaison α peut être comprise entre 20 et 60°, et de préférence voisine de 45°.

Dans une variante de mise en oeuvre avantageuse, les évidements 16a définissant les arêtes saillantes 165 sont réalisés dans une pièce biodégradable rapportée sur le support 16. La matière utilisée pour réaliser cette pièce rapportée peut être notamment un biopolymère bioabsorbable tel qu'un polylactide (PLA), un polyglycolide (PGA) ou un polylactide-co-glycolide (PLGA), ou toute autre matière équivalente, implantable et résorbable dans l'organisme. La fonction antidévissage sera alors assurée pendant une période prédéfinie, par exemple de 3 à 12 mois, pendant laquelle la fibrose recouvrira progressivement l'embase de la capsule. Puis, à long terme, il deviendra plus facile de retirer la capsule une fois la fonction antidévissage disparue.

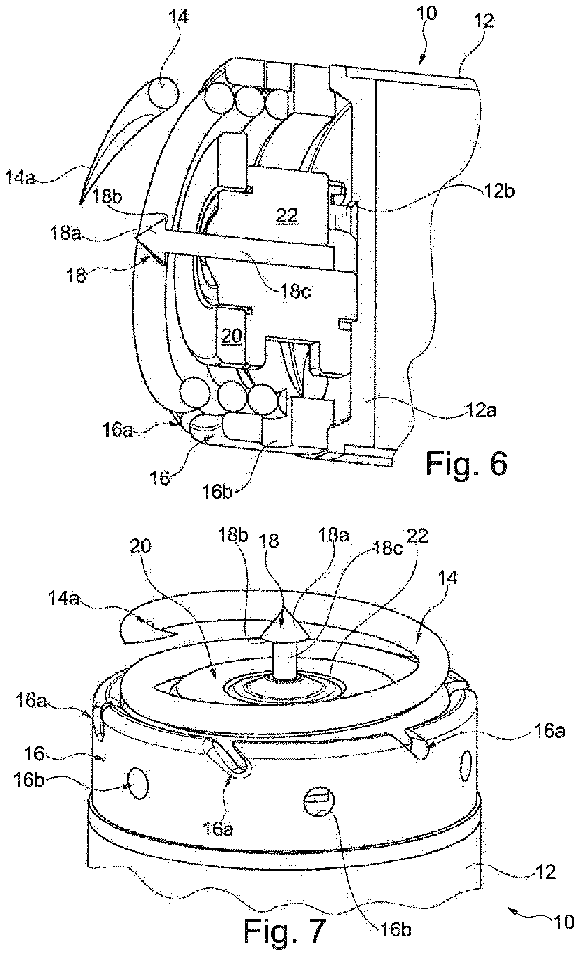

En référence maintenant aux Figures 6 et 7, on va décrire une variante de réalisation de l'électrode en pointe 18. Celle-ci présente dans ce cas une tête élargie 18a, de préférence de forme conique, rejoignant à sa base, par un épaulement annulaire rentrant 18b, une tige plus étroite 18c. L'épaulement 18 b offre une surface qui renforce mécaniquement la stabilité de la capsule sur la paroi cardiaque, en participant à la retenue des tissus comprimés par l'effort axial de la vis 14.

Dans le présent exemple, l'épaulement 18b présente une largeur annulaire de l'ordre de 0,2 mm. Cette valeur peut varier selon les besoins et les dimensions de la capsule, typiquement entre 0.05 et 1 mm.

L'angle du cône est quant à lui compris entre environ 30 et 60°, et typiquement voisin de 50°.

On va maintenant décrite une solution d'assemblage permanent de la vis 14 sur son support 16, cette solution pouvant être mise en œuvre indépendamment des caractéristiques d'antidévissage et d'accrochage à la paroi cardiaque telles que détaillées dans ce qui précède.

Le problème que résout cette solution est de pouvoir assembler une vis 14 en acier inox ou autre matériau non soudable directement sur le boitier en titane de la capsule.

Ainsi, en référence aux Figures 8 et 9, le support 16 présente une configuration comprenant un ensemble d'orifices traversants 16b, ayant une section correspondant approximativement à celle des tiges ou tubes et par exemple circulaire, formés dans la paroi du support 16, ces orifices recevant des tiges ou tubes homologues 14b formés à la périphérie de l'embase de la vis 14, dans des emplacements angulaires correspondants. Dans le présent exemple, on a prévu deux orifices traversants 16b, espacés par exemple de 60°, et deux tiges ou tubes correspondants 14b. Plus précisément, on glisse sous léger serrage d'abord la vis 14 dans son support 16 jusqu'en butée axiale contre un épaulement formé dans l'alésage central du support. Plusieurs orifices traversants radiaux 16b sont aménagés dans le support 16, qui coïncident avec la partie de la vis à spire jointive. Une tige ou un tube 14b du même matériau que la vis, ou en un matériau soudable par laser au matériau de la vis, est alors inséré dans chacun de ces trous latéraux. Dans le cas (préféré) d'un tube, l'alésage des tubes débouche alors sur les spires de la vis. Un tir laser à travers ces trous permet alors une soudure directe tube/vis assurant les conditions requises d'une bonne soudure laser, à savoir : i) compatibilité des matériaux, ii) contact direct et iii) accès visuel pour le tir et le contrôle de qualité.

Cette soudure effectuée, la vis se trouve bloquée en translation et rotation dans son support, par ancrage mécanique et sans soudure laser directe, ce qui laisse une grande liberté dans la combinaison de matériaux support/vis. Les autres avantages sensibles de cette solution sont le faible encombrement et la très faible complexité des usinages à réaliser.

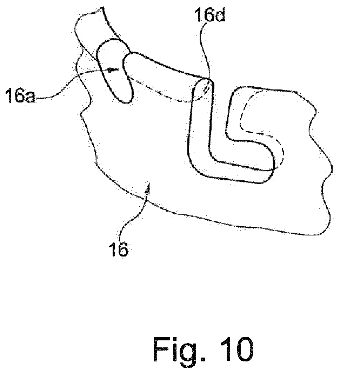

Selon une variante de réalisation illustrée sur la Figure 10 des dessins, on peut prévoir entre la vis 14 et son support 16 un montage de type baïonnette, la vis 14 comportant alors au niveau de sa base deux tiges ou tubes tels que 14b, de préférence diamétralement opposés, et le support 16 comportant dans deux régions diamétralement opposées des encoches 16d en forme générale de L s'étendant à partir de la face libre du support 16 et permettant, avant le soudage par tir laser, le montage de la vis 14 par translation axiale pour l'engagement des tiges ou tubes dans les encoches, suivie d'une rotation pour le verrouillage des tiges ou tubes au fond des encoches. Il peut alors exister, outre ces deux encoches, d'autres encoches 16d et/ou d'autres orifices circulaires 16b.

Dans le premier cas décrit plus haut, le procédé d'assemblage de la capsule comprend de préférence les étapes suivantes :
a) on fixe le support 16 au corps, par soudage,
b) on engage la base de l'élément 14 à l'intérieur dudit support,
c) on engage dans les orifices 16b les tiges ou tubes 14b,
d) on soude les tiges ou tubes sur la base de l'élément 14, par tir laser via l'intérieur du support, et
e) on soude les tiges ou tubes dans leurs orifices respectifs, par tir laser extérieurement au support.

On notera que le "soudage" de l'étape d) ne doit pas être entendu au sens étroit d'une soudure mécanique avec fusion de la matière de deux pièces distinctes, mais d'une opération destinée à effondrer la matière de la tige ou du tube à l'intérieur du logement supportant les reprises mécaniques et renforcer la fonction atraumatique par suppression des formes saillantes.

Dans le cas de la variante de la Figure 10, le procédé comprend de préférence les étapes suivantes :
a) on fixe le support 16 pourvu des encoches 16d au corps, par soudage,
b) on soude les tiges ou tubes sur la base de l'élément 14, par tir laser,
c) on pré-assemble l'élément d'ancrage par sa base dans le support, par montage de type baïonnette mettant en jeu lesdits tiges ou tubes et lesdites encoches, et
d) on soude les tiges ou tubes dans les encoches, par tir laser extérieurement au support.

Avantageusement, on prévoit des orifices 16b ou 16d en nombre supérieur au nombre de tiges ou tubes 14b de la vis 14. De la sorte, les orifices 16b ou 16d restant libres permettent de faciliter la diffusion du produit stéroïde délivré par l'anneau 24 radialement en direction de la paroi cardiaque.

Selon une autre variante, on peut prévoir que les encoches 16d prévues pour un tel montage de type baïonnette soient formées de façon à participer à une fonction d'antidévissage comme décrit précédemment.

Les différents éléments et parties décrits ci-dessus peuvent être réalisés par usinage ou autre mise en forme selon des techniques conventionnelles.

Par ailleurs, on peut prévoir comme décrit plus haut que le support 16 entoure la base de l'élément d'ancrage, mais alternativement que la base de l'élément d'ancrage entoure le support, auquel cas l'homme du métier saura faire les adaptations nécessaires, notamment quant au positionnement des tiges ou tubes 14b assurant la fixation entre l'élément d'ancrage et le support.

La capsule peut être posée par le praticien selon la technique décrite notamment dans le EP 2 394 695 A1, et extraite également selon une technique connue, la partie proximale 12 du corps de la capsule étant aménagée de façon adaptée.

L'invention ne se limite nullement à la fixation d'une capsule de stimulation autonome dans une paroi cardiaque, mais peut être mise en œuvre pour d'autres systèmes implantables, qu'ils soient autonomes ou en bout de sonde, du corps humain. En fonction de la nature de la paroi de fixation visée, les longueurs d'hélice et le cas échéant d'électrode peuvent seront facilement adaptées par l'homme du métier, sans pour autant dégrader les performances de fixation et d'irréversibilité.

## Revendications

1. Capsule implantable (10), notamment capsule autonome de stimulation cardiaque, comprenant un corps tubulaire (12) pourvu à son extrémité distale d'un élément d'ancrage à vis hélicoïdale (14) apte à pénétrer dans un tissu d'une paroi d'un organe d'un patient, le corps abritant un ensemble d'éléments fonctionnels de la capsule, la capsule comprenant un ensemble de pointes orientées dans une direction circonférentielle opposée à celle du vissage,
**caractérisée en ce qu'**elle comprend :
- une face d'appui annulaire (16c) formée dans une région annulaire entourant la base de la vis et située à l'extérieur de l'organe d'ancrage (14) en direction radiale ; et
- formés de manière affleurante dans ladite face d'appui annulaire (16c), des aménagements en creux (16a) définissant ledit ensemble de pointes (165).

2. Capsule selon la revendication 1, **caractérisée en ce que** les aménagements en creux (16a) comprennent des évidements obliques formés à partir d'une face annulaire généralement continue (16c) dudit support (16) de façon à former des pointes dirigées dans une direction circonférentielle opposée au vissage.

3. Capsule selon la revendication 2, **caractérisée en ce que** l'angle d'inclinaison des évidements est compris entre 40 et 60° par rapport à la surface de ladite face annulaire.

4. Capsule selon la revendication 1, **caractérisée en ce que** la proportion de la superficie de la face annulaire (16c) occupée par les évidements (16a) est comprise entre 20 et 40 %, et préférentiellement voisine de 30 %.

5. Capsule selon la revendication 1, **caractérisé en ce que** l'organe d'ancrage à vis (14) est formé à partir d'un fil métallique élastique enroulé en hélice et se terminant en pointe (14a) et apte à générer un effort de traction de ladite région annulaire en direction de ladite paroi.

6. Capsule selon la revendication 5, **caractérisée en ce que** l'espacement entre les spires successives de l'hélice (14) augmente entre une base de la vis et sa pointe.

7. Capsule selon la revendication 1, **caractérisée en ce qu'**elle comprend en outre une électrode en pointe (18) en saillie à l'intérieur de l'organe à vis, ladite électrode étant configurée en harpon.

8. Capsule selon la revendication 7, **caractérisée en ce que** ladite électrode (18) est une pièce de révolution centrée sur l'axe de rotation de la capsule lors du vissage.

9. Capsule selon la revendication 8, **caractérisée en ce que** ladite électrode (18) comprend une tête conique (18a) à l'extrémité libre d'une tige (18c) plus étroite que ladite tête.

10. Capsule selon la revendication 1, comprenant en outre un anneau (24) de diffusion de produit pharmaceutique, disposé à l'intérieur de ladite région annulaire (16c), lesdits évidements (16a) étant aptes à favoriser la circulation du produit vers l'extérieur de ladite région.

11. Capsule selon la revendication 1, **caractérisée en ce qu'**elle comprend une pièce en matériau biodégradable dans laquelle sont réalisés les aménagements en creux (16a) définissant ledit ensemble de pointes (165).

12. Capsule selon la revendication 11, **caractérisée en ce que** ledit matériau biodégradable est un biopolymère bioabsorbable du groupe formé par : les polylactides PLA, les polyglycolides PGA et les polylactides-co-glycolides PLGA.

## Patentansprüche

1. Implantierbare Kapsel (10), insbesondere in sich geschlossene Herzstimulationskapsel, die einen röhrenförmigen Körper (12) umfasst, der an seinem distalen Ende mit einem schraubenförmigen Schrauben-verankerungselement (14) versehen ist, das in ein Gewebe einer Wand eines Organs eines Patienten eindringen kann, wobei der Körper eine Gruppe von Funktionselementen der Kapsel aufnimmt, wobei die Kapsel eine Gruppe von Spitzen umfasst, die in einer Umfangsrichtung entgegengesetzt zu der der Verschraubung ausgerichtet sind, **dadurch gekennzeichnet, dass** sie umfasst:
- eine ringförmige Stützfläche (16c), die in einem ringförmigen Bereich ausgebildet ist, der die Schraubenbasis umgibt und sich radial außerhalb des Verankerungselements (14) befindet; und
- die bündig in der ringförmigen Lagerfläche (16c) ausgebildet sind, wobei vertiefte Anordnungen (16a) den Satz von Spikes (165) definieren.

2. Kapsel nach Anspruch 1, **dadurch gekennzeichnet, dass** die ausgesparten Anordnungen (16a) schräge Ausnehmungen aufweisen, die von einer im Allgemeinen durchgehenden Ringfläche (16c) des Trägers (16) gebildet werden, um so Spitzen zu bilden, die in einer der Verschraubung entgegengesetzten Umfangsrichtung gerichtet sind.

3. Kapsel nach Anspruch 2, **dadurch gekennzeichnet, daß** der Neigungswinkel der Ausnehmungen zwischen 40 und 60° in bezug auf die Oberfläche der ringförmigen Fläche beträgt.

4. Kapsel nach Anspruch 1, **dadurch gekennzeichnet, dass** der Anteil der von den Vertiefungen (16a) eingenommenen Fläche der Ringfläche (16c) zwischen 20 und 40%, vorzugsweise nahe 30%, liegt.

5. Kapsel nach Anspruch 1, **dadurch gekennzeichnet, dass** der Schraubanker (14) aus einem gewickelten elastischen Draht schraubenförmig gebildet ist und in einem Punkt (14a) endend und in der Lage ist, eine Zugkraft von dem ringförmigen Bereich in Richtung der Wand zu erzeugen.

6. Kapsel nach Anspruch 5, **dadurch gekennzeichnet, dass** der Abstand zwischen aufeinanderfolgenden Windungen der Wendel (14) zwischen einer Basis der Schraube und ihrer Spitze zunimmt.

7. Kapsel nach Anspruch 1, **dadurch gekennzeichnet, dass** sie ferner eine spitze Elektrode (18) umfasst, die in das Innere des Schraubenelements hineinragt, wobei die Elektrode als Harpune konfiguriert ist.

8. Kapsel nach Anspruch 7, **dadurch gekennzeichnet, dass** die Elektrode (18) ein Drehteil ist, das während des Schraubens auf der Drehachse der Kapsel zentriert ist.

9. Kapsel nach Anspruch 8, **dadurch gekennzeichnet, dass** die Elektrode (18) einen konischen Kopf (18a) am freien Ende eines Schaftes (18c) aufweist, der schmaler als der Kopf ist.

10. Kapsel nach Anspruch 1, die ferner einen Ring (24) zur Diffusion eines pharmazeutischen Produkts aufweist, der innerhalb des ringförmigen Bereichs (16c) angeordnet ist, wobei die Ausnehmungen (16a) geeignet sind, die Zirkulation des Produkts zur Außenseite des Bereichs zu fördern.

11. Kapsel nach Anspruch 1, **dadurch gekennzeichnet, dass** sie ein Teil aus biologisch abbaubarem Material umfasst, in dem die hohlen Anordnungen (16a), die den Satz von Spitzen (165) definieren, hergestellt sind.

12. Kapsel nach Anspruch 11, **dadurch gekennzeichnet, dass** das biologisch abbaubare Material ein bioabsorbierbares Biopolymer aus der Gruppe bestehend aus: PLA-Polylactiden, PGA-Poiyglykoliden und PLGA-Polylactidco-Glykoliden ist.

## Claims

1. Implantable capsule (10), in particular a self-contained cardiac stimulation capsule, comprising a tubular body (12) provided at its distal end with a helical screw anchoring element (14) capable of penetrating into a tissue of a wall of an organ of a patient, the body housing a set of functional elements of the capsule, the capsule comprising a set of spikes oriented in a circumferential direction opposite to that of the screwing, **characterised in that** it comprises :
- an annular support face (16c) formed in an annular region surrounding the screw base and located radially outwardly of the anchor member (14); and
- formed flush in said annular bearing face (16c), recessed arrangements (16a) defining said set of spikes (165).

2. The capsule according to claim 1, **characterised in that** the recessed arrangements (16a) comprise oblique recesses formed from a generally continuous annular face (16c) of said support (16) so as to form tips directed in a circumferential direction opposite to the screwing.

3. The capsule according to claim 2, **characterised in that** the angle of inclination of the recesses is between 40 and 60° with respect to the surface of said annular face.

4. The capsule according to claim 1, **characterised in that** the proportion of the area of the annular face (16c) occupied by the recesses (16a) is between 20 and 40%, and preferably close to 30%.

5. The capsule according to claim 1, **characterised in that** the screw anchor (14) is formed helically from a wound elastic wire and terminating in a point (14a) and capable of generating a tensile force from said annular region towards said wall.

6. The capsule according to claim 5, **characterised in that** the spacing between successive turns of the helix (14) increases between a base of the screw and its tip.

7. The capsule according to claim 1, **characterised in that** it further comprises a pointed electrode (18) projecting inside the screw member, said electrode being configured as a harpoon.

8. The capsule according to claim 7, **characterised in that** said electrode (18) is a piece of revolution centred on the axis of rotation of the capsule during screwing.

9. The capsule according to claim 8, **characterised in that** said electrode (18) comprises a conical head (18a) at the free end of a stem (18c) narrower than said head.

10. The capsule according to claim 1, further comprising a ring (24) for diffusing a pharmaceutical product, disposed inside said annular region (16c), said recesses (16a) being capable of promoting the circulation of the product towards the outside of said region.

11. The capsule according to claim 1, **characterised in that** it comprises a piece of biodegradable material in which the hollow arrangements (16a) defining said set of tips (165) are made.

12. The capsule according to claim 11, **characterized in that** said biodegradable material is a bioabsorbable biopolymer from the group consisting of: PLA polylactides, PGA poiyglycolides and PLGA polylactide-co-glycolides.
